(19) Europäisches Patentamt
European Patent Office
Office européen des brevets

(11) Veröffentlichungsnummer: **0 276 710 B1**

(12) **EUROPÄISCHE PATENTSCHRIFT**

(45) Veröffentlichungstag der Patentschrift: **27.12.91**

(51) Int. Cl.⁵: **C07D 413/04**, C07D 413/12, C07D 271/04, A61K 31/41

(21) Anmeldenummer: **88100574.8**

(22) Anmeldetag: **16.01.88**

Die Akte enthält technische Angaben, die nach dem Eingang der Anmeldung eingereicht wurden und die nicht in dieser Patentschrift enthalten sind.

# (54) Allylmercaptoacetyl-sydnonimine, Verfahren zu ihrer Herstellung und ihre Verwendung.

(30) Priorität: **24.01.87 DE 3702083**

(43) Veröffentlichungstag der Anmeldung:
**03.08.88 Patentblatt 88/31**

(45) Bekanntmachung des Hinweises auf die Patenterteilung:
**27.12.91 Patentblatt 91/52**

(84) Benannte Vertragsstaaten:
**AT BE CH DE ES FR GB GR IT LI NL SE**

(56) Entgegenhaltungen:
**EP-A- 0 076 952**
**DE-A- 2 930 736**
**DE-A- 3 107 933**
**DE-A- 3 526 068**

**CHEMICAL ABSTRACTS, Band 101, Nr. 15, 8. Oktober 1984, Columbus, Ohio, USA; KUKOVETZ, W.R.; HOLZMANN, S.; STRAKA, M; SCHMIDT, K. "Mechanism of the blood vessel dilation effect of molsidomine" Seite 34, Spalte 2, Zusammenfassung-Nr. 122 739x**

(73) Patentinhaber: **CASSELLA Aktiengesellschaft**
**Hanauer Landstrasse 526**
**W-6000 Frankfurt am Main 61(DE)**

(72) Erfinder: **Schönafinger, Karl, Dr.**
**Holunderweg 8**
**W-8755 Alzenau(DE)**
Erfinder: **Beyerle, Rudi, Dr.**
**An der Pfaffenmauer 44**
**W-6000 Frankfurt 60(DE)**
Erfinder: **Bohn, Helmut, Dr.**
**Kranzbergring 11**
**W-6369 Schöneck(DE)**
Erfinder: **Just, Melitta, Dr.**
**Hüttenberg 6**
**W-6369 Schöneck(DE)**
Erfinder: **Nitz, Rolf-Eberhard, Dr.**
**Heinrich-Bingemer-Weg 64**
**W-6000 Frankfurt 60(DE)**

(74) Vertreter: **Urbach, Hans-Georg, Dr. et al**
**Hanauer Landstrasse 526**
**W-6000 Frankfurt am Main 61(DE)**

**EP 0 276 710 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTRACTS, Band 101, Nr. 15, 8. Oktober 1984, Columbus, Ohio, USA; BOEHME, E.; SPIES, C; GROSSMANN, G. "Active metabolite of molsidomine and stimulation of cGMP-formation with sydnonimine" Seite 35, Spalte 1, Zusammenfassung-Nr. 122 740r

## Beschreibung

Die Erfindung betrifft pharmakologisch wirksame, substituierte Allylmercaptoacetyl-sydnonimine der allgemeinen Formel I

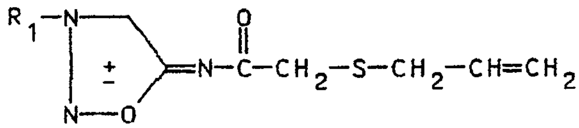

(I)

worin $R_1$ für eine sekundäre Aminogruppe der Formel

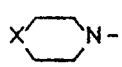 oder 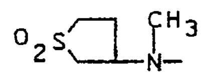

oder $-N(R_3)_2$ steht und X $NR_2$, $S(O)_n$, O oder $(CH_2)_n$,
$R_2$ einen der Reste $R_3OOC$-, $R_4SO_2$-, oder Alkyl mit 1 - 4 C-Atomen,
n einen der Werte 0, 1 oder 2,
und $R_3$ Alkyl mit 1-4 C-Atomen bedeuten
und $R_4$ für $R_3$ oder $(R_3)_2N$- steht,
sowie deren pharmakologisch unbedenklichen Salze.

Die Erfindung betrifft weiterhin Verfahren zur Herstellung dieser Allylmercaptoacetyl-sydnoniminderivate und ihrer Salze sowie deren Verwendung als pharmazeutische Wirkstoffe.

Bevorzugte Verbindungen der Formel I sind solche, bei denen
$R_1$ den Rest

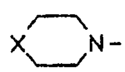

X $NR_2$, $SO_2$ oder O, und
$R_2$ $R_3OOC$- oder $R_4SO_2$- und
$R_3$ Methyl oder Ethyl und
$R_4$ Methyl, Ethyl, Dimethylamino oder Diethylamino bedeuten.

Bevorzugte Reste $R_1$ sind insbesondere:

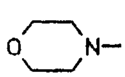 , 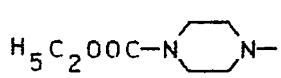 , 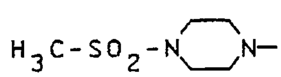 , 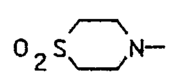 ,

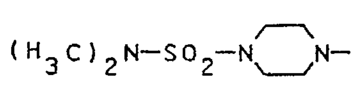 , 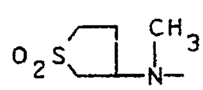 , 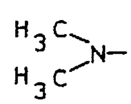 ,

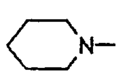 , 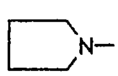 .

Besonders bevorzugt für $R_1$ ist der Morpholinorest. Die für $R_3$ und $R_4$ stehenden Alkylreste können geradkettig oder verzweigt sein.

Die erfindungsgemäßen Allylmercaptoacetyl-sydnonimine der allgemeinen Formel I können nach verschiedenen an sich bekannten Verfahren erhalten werden. Diese Verfahren sind dadurch gekennzeichnet, daß man

a) ein Sydnonimin der allgemeinen Formel II

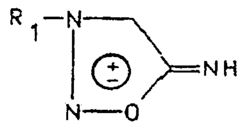 (II)

gegebenenfalls in Form eines Salzes mit einem den Allylmercaptoacetylrest einführenden Acylierungsmittel umsetzt oder

b) ein Sydnonimin der allgemeinen Formel II gegebenenfalls in Form eines Salzes mit einem Essigsäurederivat acyliert, das einen Rest enthält, der mit Allylmercaptan anschließend zur Allylmercapto-Verbindung der allgemeinen Formel I umgesetzt wird.

Die Herstellung der als Ausgangsstoffe eingesetzten Verbindungen der Formel II

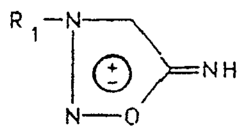 (II)

worin $R_1$ die genannten Bedeutungen besitzt, ist beispielsweise beschrieben in den europäischen Patentschrif ten 23343 und 59356.

Die Acylierung der Verbindungen der Formel II zur Einführung der Reste

$$-\overset{\overset{\displaystyle O}{\|}}{C}-CH_2-S-CH_2-CH=CH_2 \quad \text{bzw.} \quad -\overset{\overset{\displaystyle O}{\|}}{C}-CH_2-Z,$$

wobei Z eine gegen den Rest $-S-CH_2-CH=CH_2$ austauschbare Gruppe bedeutet, kann in an sich bekannter Weise mit geeigneten Acylierungsmitteln der Formeln IIIa und b

$$X-\overset{\overset{\displaystyle O}{\|}}{C}-CH_2-S-CH_2-CH=CH_2 \quad \text{bzw.} \quad X-\overset{\overset{\displaystyle O}{\|}}{C}-CH_2-Z, \qquad \text{(IIIa und b)}$$

worin X z.B. Halogen, insbesondere Chlor, Aryloxy, insbesondere Tolyloxy, Dinitrophenyloxy oder Nitrophenyloxy oder O-Acyl mit dem gleichen Acylrest (d.h. einem Säureanhydrid) bedeutet, durchgeführt werden. Z kann insbesondere Halogen, $OSO_2CH_3$, $OSO_2Ar$ bedeuten, wobei Ar für einen gegebenenfalls substituierten Phenylrest steht.

Die Umsetzung zwischen dem Acylierungsmittel und der Verbindung II wird zweckmäßigerweise in flüssiger Phase in Anwesenheit eines inerten Lösungs-, Dispergier- oder Verdünnungsmittels durchgeführt.

Geeignete Lösungs-, Dispergier- oder Verdünnungsmittel sind z.B. Alkohole, insbesondere solche mit 1 bis 6 C-Atomen, wie z.B. Methanol, Ethanol, i- und n-Propanol, i-, sec- und tert-Butanol, n-, i-, sec-, tert-Pentanol, n-Hexanol, Cyclopentanol, Cyclohexanol; Ether, insbesondere solche mit 2 bis 8 C-Atomen im Molekül, wie z.B. Diethylester, Methyl-ethyl-ether, Di-n-propyl-ether, Di-isopropyl-ether, Methyl-n-butyl-ether, Ethylpropylether, Di-butyl-ether, Tetrahydrofuran, 1,4-Dioxan , 1,2-Dimethoxyethan, Bis-$\beta$-methoxyethylether; Polyether, wie z.B. Polyethylenglykole mit einem Molekulargewicht bis ca. 600; Cligoethylenglycol-dimethyl-ether, wie z.B. Pentaglyme; Kronenether, d.h. cyclische Polymere des Ethylenglykols der Formel $(-OCH_2CH_2)_p$, wobei p eine Zahl z.B. von 4 bis 10 ist, wobei an den Ring auch eine oder mehrere Benzolringe ankondensiert sein können; Aza- und Thia-kronenether (auch als Coronand-amine bzw. Coronandsulfide bezeichnet);

Glykole und teilweise veretherte Glykole, wie z.B. Ethylenglykol, Propylenglykol, Trimethylenglykol, Ethylenglykol-monomethyl-ether, Ethylenglykol-monoethyl-ether, Di-ethylenglykol-monoethyl-ether; aliphatische Kohlenwasserstoffe, wie z.B. Benzine, niedrig- und hochsiedende Petrolether; aromatische Kohlenwasserstoffe, wie z.B. Benzol, Toluol, o-, m-und p-Xylol, Pyridin; halogenierte aliphatische oder aromatische Kohlenwasserstoffe, wie z.B. Methylenchlorid, Chloroform, Tetrachlorkohlenstoff, Ethylenchlorid, Chlorbenzol, Dichlorbenzol; Nitrile, wie z.B. Acetonitril; Amide, wie z.B. Dimethylformamid, N-Methyl-pyrrolidon;

Sulfoxide, wie z.B. Dimethyl-sulfoxid; Wasser. Auch Gemische verschiedener Lösungs-, Dispergier- oder Verdünnungsmittel können verwendet werden, z.B. Wasser/Methylenchlorid, Wasser/Toluol. Auch ein Überschuß des Acylierungsmittels kann als Lösungs-, Dispergier- oder Verdünnungsmittel verwendet werden.

Die als Lösungs-, Dispergier- oder Verdünnungsmittel angegebenen Alkohole, Glykole und teilweise veretherten Glykole sowie Wasser sind normalerweise nur für die Acylierung mit Carbonsäureestern geeignet, dagegen für die Durchführung der Acylierung mit anderen Acylierungsmitteln wegen der konkurrierenden Bildung von Estern, Glykolestern oder Säuren nicht ausreichend inert und daher weniger geeignet.

Das Molverhältnis zwischen der Verbindung der Formel II und dem Acylierungsmittel beträgt 1 : 1. Zweckmäßigerweise wird das Acylierungsmittel in einem geringen molaren Überschuß eingesetzt. Überschüsse von bis zu 30 mol% sind in der Regel ausreichend, d.h. das Molverhältnis zwischen der Verbindung der Formel II und dem Acylierungsmittel beträgt normalerweise 1 : (1 bis 1,3), vorzugsweise 1 : (1 bis 1,2). Falls bei der Acylierungsreaktion eine Säure abgespalten wird, ist der Zusatz eines Säurefängers, wie z.B. eines Alkalihydroxids, wie z.B. Natrium-, Kalium-oder Lithium-hydroxids, eines tertiären organischen Amins, wie z.B. Pyridin oder Triethylamin, eines Alkalicarbonats oder Alkalibicarbonats, wie z.B. Soda oder Natriumbicarbonat, oder eines Alkalisalzes einer Schwachen organischen Säure, wie z.B. Natriumacetat, zweckmäßig. Bei der Acylierungsreaktion können auch geeignete Katalysatoren, wie z.B. 4-Dimethylaminopyridin, zugesetzt werden.

Die Umsetzung zwischen dem Acylierungsmittel und der Verbindung II kann prinzipiell bei Temperaturen zwischen -10 °C und dem Siedepunkt des verwendeten Lösungs-, Dispergier- oder Verdünnungsmittels erfolgen. In vielen Fällen wird die Umsetzung bei 0 bis 50 °C, insbesondere bei 0 bis 30 °C und vorzugsweise bei Raumtemperatur, durchgeführt.

Die substituierten Allylmercaptoacetyl-sydnonimine der allgemeinen Formel I bilden mit anorganischen oder organischen Säuren Säureadditionssalze. Zur Bildung derartiger Säureadditionssalze sind anorganische und organische Säuren geeignet. Geeignete Säuren sind beispielsweise Chlorwasserstoff, Bromwasserstoff, Naphthalindisulfonsäuren, insbesondere Naphthalindisulfonsäure(1,5), Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-, Isonicotin-, Methansulfon, p-Toluolsulfon-, Zitronen- oder Adipin-Säure. Pharmakologisch annehmbare Säureadditionssalze werden bevorzugt. Die Säureadditionssalze können wie üblich durch Vereinigung der Komponenten, zweckmäßigerweise in einem geeigneten Lösungs- oder Verdünnungsmittel, hergestellt werden. Bei der Synthese der Verbindungen der Formel I können diese in Form der Säureadditionssalze anfallen. Aus den Säureadditionssalzen können die freien Verbindungen der allgemeinen Formel I gewünschtenfalls in bekannter Weise, d.h. durch Auflösen oder Suspendieren in Wasser und Alkalischstellen, z.B. mit Natronlauge, und anschließendes Isolieren, gewonnen werden.

Die benötigten Ausgangsverbindungen der allgemeinen Formel II können in an sich bekannter Weise nach der Strecker'schen Aminonitrilsynthese aus Verbindungen der allgemeinen Formel

$R^1$-$NH_2$

durch Umsetzung mit Formaldehyd und Blausäure bzw. Natriumcyanid in einem geeigneten Lösungsmittel, z.B. Wasser, hergestellt werden, wobei zunächst eine Verbindung der allgemeinen Formel

$R^1$-NH-$CH_2$-CN

entsteht, die anschließend einer Nitrosierung unterworfen wird. Die Nitrosierung wird in bekannter Weise in einem geeigneten Lösungsmittel, vorzugsweise in Wasser, bei Temperaturen von 0 bis 10 °C durchgeführt. Die salpetrige Säure wird dabei normalerweise aus einem Alkalimetallnitrit und Salzsäure erzeugt. Es ist zweckmäßig, die wäßrige Lösung der Verbindung der Formel $R^1$-NH-$CH_2$-CN mit Salzsäure auf einen pH-Wert von 1 bis 3 einzustellen und das Alkalimetallnitrit in Form einer wäßrigen Lösung zu der gerührten und abgekühlten Lösung der Verbindung zuzutropfen.

Die Lösung der erhaltenen Nitrosoverbindung kann direkt der Cyclisierungsreaktion unterworfen werden. Normalerweise ist es jedoch angebracht, die Nitrosoverbindung zunächst in einem geeigneten organischen Lösungsmittel aufzunehmen und in ihm, gegebenenfalls nach Zusatz eines weiteren Lösungsmittels, die Cyclisierung zu der Verbindung der Formel II durchzuführen.

Die Verbindungen der allgemeinen Formel I und ihre pharmakologisch annehmbaren Säureadditionssalze besitzen wertvolle pharmakologische Eigenschaften.

Besonders ausgeprägt ist ihre Wirkung auf das Herz-Kreislauf-System. Verglichen mit der im Handel

befindlichen strukturähnlichen Verbindung Molsidomin wirken sie in niedrigeren Dosen und über einen noch längeren Zeitraum.

Die Verbindungen der Formel I und ihre pharmakologisch annehmbaren Säureadditionssalze können daher am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der Formel I oder eines Säureadditionssalzes davon neben üblichen pharmazeutisch einwandfreien Träger- und Zusatzstoffen enthalten.

Die Heilmittel können oral, z.B. in Form von Pillen, Tabletten, Lacktabletten, Dragees, Hart- und Weichgelatinekapseln, Lösungen, Sirupen, Emulsionen oder Suspensionen oder Aerosolmischungen verabreicht werden. Die Verabreichung kann aber auch rektal, z.B. in Form von Suppositorien, oder parenteral, z.B. in Form von Injektionslösungen, oder perkutan, z.B. in Form von Salben oder Tinkturen, erfolgen.

Zur Herstellung der pharmazeutischen Präparate können pharmazeutisch inerte anorganische oder organische Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen und Sirupen eignen sich z.B. Wasser, Saccharose, Inverzucker, Glukose, Polyole etc. Als Trägerstoffe für die Herstellung von Injektionslösungen eignen sich z.B. Wasser, Alkohole, Glyzerin, Polyole, pflanzliche Öle etc.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-, Dickungs-, Verdünnungs-Mittel, Puffersubstanzen, ferner Lösungsmittel oder Lösungsvermittler oder Mittel zur Erzielung eines Depoteffektes, sowie Salze zur Veränderung des osmotischen Drucks, Überzugsmittel oder Antioxidantien enthalten. Sie können auch zwei oder mehrere Verbindungen der Formel I oder ihrer pharmakologisch annehmbaren Säureadditionssalze und noch andere therapeutisch wirksame Stoffe enthalten.

Derartige andere therapeutisch wirksame Substanzen sind beispielsweise: β-Rezeptorenblocker, wie z.B. Propranolol, Pindolol, Metoprolol; Vasodilatatoren, wie z.B. Carbochromen; Beruhigungsmittel, wie z.B. Barbitursäurederivate, 1,4-Benzodiazepine und Meprobamat; Diuretica, wie z.B. Chlorothiazid; das Herz tonisierende Mittel, wie z.B. Digitalispräparate; blutdrucksenkende Mittel, wie z.B. Hydralazin, Dihydralazin, Prazosin, Clonidin, Rauwolfia-Alkaloide; Mittel, die den Fettsäurespiegel im Blut senken, wie z.B. Bezafibrat, Fenofibrat; Mittel für die Thromboseprophylaxe, wie z.B. Phenprocoumon.

Die Verbindungen der Formel I, ihre pharmakologisch annehmbaren Säureadditionssalze und pharmazeutische Präparate, welche die Verbindungen der Formel I oder ihre pharmakologisch annehmbaren Säureadditionssalze als Wirkstoffe enthalten, können am Menschen bei der Bekämpfung bzw. Vorbeugung von Erkrankungen des kardiovaskulären Systems verwendet werden, beispielsweise als antihypertensive Heilmittel bei den verschiedenen Formen des Bluthochdrucks, bei der Bekämpfung und zur Vorbeugung von Angina pectoris. Die Dosierung kann innerhalb weiter Grenzen variieren und ist in jedem einzelnen Fall den individuellen Gegebenheiten anzupassen. Im allgemeinen ist bei oraler Verabreichung pro menschlichem Individuum eine Tagesdosis von etwa 0,5 bis 100 mg, vorzugsweise 1 bis 20 mg, angemessen. Auch bei anderen Applikationsformen liegt die Tagesdosis wegen der guten Resorption der Wirkstoffe in ähnlichen Mengenbereichen, d.h. im allgemeinen ebenfalls bei 0,5 bis 100 mg/Mensch. Die Tagesdosis wird normalerweise in mehrere, z.B. 2 bis 4 Teilverabreichungen aufgeteilt.

Die folgenden Beispielen dienen der näheren Erläuterung der Erfindung.

Beispiel 1
3-Morpholino-N-allylmercaptoacetyl-sydnonimin

2 g Allylmercaptan werden in 50 ml Methanol gelöst, und unter Stickstoff mit 6 g 3-Morpholino-N-chloracetylsydnonimin versetzt. Eine Lösung von 2,7 g Kalium-tert.butylat in 20 ml Methanol wird zugetropft und die Mischung 12 Stunden bei Raumtemperatur unter Stickstoff gerührt.

Nach Abfiltrieren eines unlöslichen Nebenprodukts wird die Reaktionslösung im Vakuum eingeengt, der Rückstand in Essigester aufgenommen, erneut filtriert und wieder eingeengt. Der dabei erhaltene Rückstand wird schließlich mit Ether verrührt, abgesaugt und getrocknet.

Ausbeute: 4,0 g 3-Morpholino-N-allylmercaptoacetyl-sydnonimin;
Fp = 83-84 °C

Beispiel 2
3-(4-Ethoxycarbonyl-piperazin-1-yl)-N-allylmercaptoacetyl-sydnonimin

Eine Lösung von 5,6 g 3-(4-Ethoxicarbonyl-piperazin-1-yl)-sydnonimin-hydrochlorid in 50 ml Wasser wird auf 5° C abgekühlt. 4.2 g festes Natriumhydrogencarbonat werden zugefügt und das Reaktionsgemisch mit einer Lösung von 3,8 g Allylmercaptoacetylchlorid in 50 ml Methylenchlorid versetzt. Die Mischung wird 3 Stunden bei Raumtemperatur gerührt. Die organische Phase wird abgetrennt und über Natriumsulfat getrocknet und am Rotationsverdampfer eingeengt. Der Rückstand wird mit Ether verrührt. Der erhaltene Feststoff wird abgesaugt und aus Essigester umkristallisiert.

Ausbeute: 5,9 g 3-(4-Ethoxicarbonyl-piperazin-1-yl)-N-allylmercaptoacetyl-sydnonimin;
Fp = 96-97° C

In analoger Weise wie in den Beispielen 1 und 2 beschrieben, werden nachfolgende Produkte hergestellt:

Beispiel 3
3-(4-Methylsulfonyl-piperazin-1-yl)-N-allylmercaptoacetyl-sydnonimin;

Fp = 102-103° C,

Beispiel 4
3- (4-Dimethylaminosulfonyl-piperazin-1-yl)-N-allylmercaptoacetyl-sydnonimin;

Fp = 79-80° C

Beispiel 5
3-(1.1-Dioxo-tetrahydro-1.4-thiazin-4-yl)-N-allylmercaptoacetyl-sydnonimin;

Fp = 109-110° C

Beispiel 6
3-(N-Methyl-N-1.1-dioxo-tetrahydrothien-3-yl-amino)-N-allylmercaptoacetyl-sydnonimin;

Fp = 105-106° C

Beispiel 7
3-Dimethylamino-N-allylmercaptoacetyl-sydnonimin;

Fp = 100-101° C

Beispiel 8
3-Piperidino-N-allylmercaptoacetyl-sydnonimin;

Fp = 97-98° C

Beispiel 9
3-Pyrrolidino-N-allylmercaptoacetyl-sydnonimin;

Fp = 108-109° C

Die folgenden Beispiele betreffen pharmazeutische Zubereitungen.

| Beispiel A | |
|---|---|
| Tabletten | pro Tablette |
| erfindungsgemässes 3-substituiertes N-Allylmercaptoacetyl-sydnonimin | 20 mg |
| Lactose | 60 mg |
| Maisstärke | 30 mg |
| lösliche Stärke | 5 mg |
| Magnesiumstearat | 5 mg |
| | 120 mg |

| Beispiel B | |
|---|---|
| Dragees | |
| erfindungsgemässes 3-substituiertes N-Allylmercaptoacetyl-sydnonimin | 6 mg |
| Propranolol | 40 mg |
| Milchzucker | 90 mg |
| Maisstärke | 90 mg |
| sec. Calciumphosphat | 34 mg |
| | 260 mg |

| Beispiel C | |
|---|---|
| Kapseln | |
| erfindungsgemässes 3-substituiertes N-Allylmercaptoacetyl-sydnonimin | 5 mg |
| Prazosin | 5 mg |
| Maisstärke | 185 mg |
| | 195 mg |

| Beispiel D | |
|---|---|
| Injektionslösung | |
| erfindungsgemässes 3-substituiertes N-Allylmercaptoacetyl-sydnonimin | 4 mg |
| Natriumchlorid | 0,7 mg |
| Wasser zu Injektionszwecken ad | 1 ml |

| Beispiel E | |
|---|---|
| Suppositorien | |
| erfindungsgemässes 3-substituiertes N-Allylmercaptoacetyl-sydnonimin | 20 mg |
| Suppositoriumsgrundmasse ad | 2 g |

Die pharmakologische Wirkung der Verbindung der Formel I wurde nach einer modifizierten Methode von Godfraind and Kaba (Arch. Int. Pharmacodyn. Ther. 196, (Suppl) 35 bis 49, 1972) und von Schümann et al (Naunyn-Schmiedeberg's Arch. Pharmacol. 289, 409 bis 418, 1975) ermittelt. Dabei werden Spiralstreifen der Arteria pulmonalis des Meerschweinchens nach Äquilibrierung in calciumfreier Tyrodelösung mit 40 mmol/l Kalium depolarisiert. Ein Zusatz von 0,5 mmol/l $CaCl_2$ löst dann eine Kontraktion aus. Die relaxierende Wirkung der Prüfsubstanz wird durch kumulative Zugabe in 1/2 log 10 abgestuften Konzentrationen ermittelt. Aus der Konzentrationswirkungskurve (Abszisse: -log mol/l Prüfsubstanz, Ordinate: % Hemmung der maximalen Kontraktion, Mittelwert von 4 bis 6 Gefäßstreifen) wird die Konzentration der

Prüfsubstanz ermittelt, welche die Kontraktion um 50% hemmt (= $IC_{50}$, mol/l). In der folgenden Tabelle sind die so erhaltenen $IC_{50}$-Werte angegeben. Wie der Vergleich mit dem $IC_{50}$-Wert $1 \cdot 10^{-4}$ für die bekannte Verbindung Molsidomin (N-Ethoxycarbonyl-3-morpholino-sydnonimin), vgl. DE-B-16 95 897, ergibt, liegen die Werte für die Verbindungen der Formel I erheblich günstiger.

Tabelle

| Verbindung der Formel I gemäß Beispiel | $IC_{50}$ (mol/l) |
|---|---|
| 1 | $2 \cdot 10^{-5}$ |
| 2 | $8 \cdot 10^{-5}$ |
| 3 | $3 \cdot 10^{-5}$ |
| 4 | $3 \cdot 10^{-5}$ |
| 5 | $3 \cdot 10^{-5}$ |
| 6 | $1 \cdot 10^{-5}$ |
| 7 | $3 \cdot 10^{-5}$ |
| 8 | $2 \cdot 10^{-5}$ |
| Molsidomin (N-Ethoxycarbonyl-3-morpholino-sydnonimin) (Vergleichssubstanz) | $> 1 \cdot 10^{-4}$ |

Die DE-A-3 107 933 und DE-A-2 930 736 betreffen substituierte 3-Amino-sydnonimine und die DE-A-3 522 068 betrifft substituierte 3-Amino-sydnonimine in optisch aktiver Form.

Die substituierten 3-Amino-sydnonimine der DE-A-3107933 sind in 3-Stellung z.B. durch die Reste N-Methyl-N-tetrahydro-3-thienyl-S,S-dioxid)-amino oder 4-Ethoxycarbonylpiperazin-1-yl substituiert. Diese beiden Reste können auch als Substituenten in 3-Stellung bei den Verbindungen der DE-A-3526068 neben anderen Substituenten, wie z.B. Morpholino, Piperidino, auftreten. Bei den Verbindungen der DE-A-2930736 können als Substituenten in 3-Stellung z.B. Thiomorpholino, Tetrahydro-1,4-thiazin-4-yl oder 4-Methansulfonyl-piperazin-1-yl auftreten. Die Verbindungen der DE-A-3526068 sind am $N^6$-Atom durch Acyl, z.B. Methoxy-propionyl oder Acetoxy-phenylacetyl substituiert. Auch die Verbindungen der DE-A-3107933 und DE-A-2930736 können am $N^6$-Atom durch Acyl, wie z.b. Ethoxycarbonyl, substituiert sein. Da keine der drei vorgenannten Literaturstellen Hinweise auf Alkylmercaptoacetyl als möglichen Substituenten am $N^6$-Atom enthält, werden die erfindungsgemäßen Verbindungen durch den Stand der Technik nicht nahegelegt.

## Patentansprüche

### Patentansprüche für folgende Vertragsstaaten : AT, BE, CH, DE, FR, GB, IT, NL, SE

**1.** Allylmercaptoacetyl-sydnonimine der allgemeinen Formel I

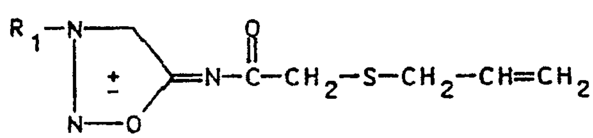

(I)

worin $R_1$ für eine sekundäre Aminogruppe der Formel

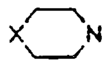 oder 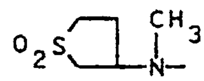

oder $N(R_3)_2$ steht
und X $NR_2$, $S(O)_n$, O oder $(CH_2)_n$,
$R_2$ einen der Reste $R_3OOC$-, $R_4SO_2$- oder Alkyl mit 1 - 4 C-Atomen,
n einen der Werte 0, 1 oder 2,
und $R_3$ Alkyl mit 1-4 C-Atomen bedeuten
und $R_4$ für $R_3$ oder $(R_3)_2N$- steht
sowie deren pharmakologisch unbedenklichen Salze.

**2.** Allylmercaptoacetyl-sydnonimine gemäß Anspruch 1, dadurch gekennzeichnet, daß $R_1$ den Rest

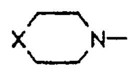

bedeutet
und X $NR_2$, $SO_2$ oder O, und $R_2$,
$R_3OOC$- oder $R_4SO_2$- und $R_3$ Methyl oder Ethyl,
und $R_4$ Methyl, Ethyl, Dimethylamino oder Diethylamino bedeutet.

**3.** Allylmercaptoacetyl-sydnonimin gemäß Anspruch 1, dadurch gekennzeichnet, daß $R_1$ die Bedeutung

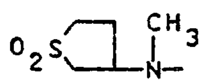

besitzt.

**4.** Allylmercaptoacetyl-sydnonimin der Formel I, dadurch gekennzeichnet, daß $R_1$

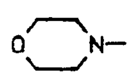 ,

bedeutet.

**5.** Verfahren zur Herstellung von Allylmercaptoacetyl-sydnonimin der in Anspruch 1 angegebenen allgemeinen Formel I, dadurch gekennzeichnet, daß man in an sich bekannter Weise
a) ein Sydnonimin der allgemeinen Formel II

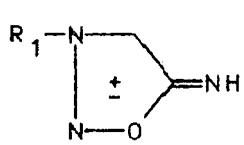 (II)

gegebenenfalls in Form eines Salzes mit einem den Allylmercaptoacetylrest einführenden Acylierungsmittel umsetzt oder
b) ein Sydnonimin der allgemeinen Formel II gegebenenfalls in Form eines Salzes mit einem Essigsäurederivat acyliert, das einen Rest enthält, der mit Allylmercaptan anschließend zur Allylmercapto-Verbindung der allgemeinen Formel I umgesetzt wird.

**6.** Allylmercaptoacetyl-sydnonimine der allgemeinen Formel I oder deren pharmakologisch unbedenklichen Säureadditionssalze als pharmakologische Wirkstoffe bei der Behandlung cardiovaskulärer Erkrankungen oder Angina pectoris.

7. Pharmazeutisches Präparat, dadurch gekennzeichnet, daß es ein Allylmercaptoacetyl-sydnonimin der allgemeinen Formel I oder ein pharmakologisch unbedenkliches Säureadditionssalz davon als Wirkstoff zusammen mit pharmazeutisch annehmbaren Träger- und Zusatzstoffen, gegebenenfalls in Kombination mit anderen pharmazeutischen Wirkstoffen, enthält.

8. Verwendung der Allylmercaptoacetyl-sydnonimine der allgemeinen Formel I oder eines pharmakologisch unbedenklichen Säureadditionssalzes davon zur Herstellung von Arzneimitteln zur Behandlung cardiovaskulärer Erkrankungen oder Angina pectoris.

**Patentansprüche für folgende Vertragsstaaten : ES, GR**

1. Verfahren zur Herstellung von Allylmercaptoacetylsydnonimine der allgemeinen Formel I

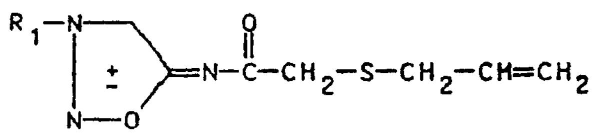

(I)

worin $R_1$ für eine sekundäre Aminogruppe der Formel

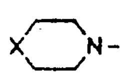

oder

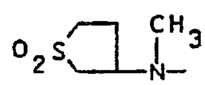

oder $-N(R_3)_2$ steht
und X $NR_2$, $S(O)_n$, O oder $(CH_2)_n$,
$R_2$ einen der Reste $R_3OOC$-, $R_4SO_2$- oder Alkyl mit 1 - 4 C-Atomen,
n einen der Werte 0, 1 oder 2,
und $R_3$ Alkyl mit 1-4 C-Atomen bedeuten
und $R_4$ für $R_3$ oder $(R_3)_2N$- steht
sowie deren pharmakologisch unbedenklichen Salze,
dadurch gekennzeichnet, daß man in an sich bekannter Weise

a) ein Sydnonimin der allgemeinen Formel II

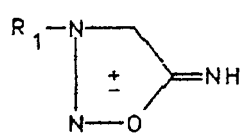

(II)

gegebenenfalls in Form eines Salzes mit einem den Allylmercaptoacetylrest einführenden Acylierungsmittel umsetzt oder

b) ein Sydnonimin der allgemeinen Formel II gegebenenfalls in Form eines Salzes mit einem Essigsäurederivat acyliert, das einen Rest enthält, der mit Allylmercaptan anschließend zur Allylmercapto-Verbindung der allgemeinen Formel I umgesetzt.

2. Verfahren nach Anspruch 1, dadurch gekennzeichnet, daß $R_1$ den Rest

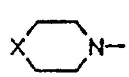

bedeutet
und X $NR_2$, $SO_2$ oder O, und $R_2$,

$R_3OOC$- oder $R_4SO_2$- und $R_3$ Methyl oder Ethyl, und $R_4$ Methyl, Ethyl, Dimethylamino oder Diethylamino bedeutet.

3. Verfahren nach Anspruch 1, dadurch gekennzeichnet, daß $R_1$ die Bedeutung

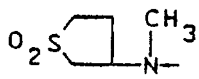

besitzt.

4. Verfahren nach Anspruch 1, dadurch gekennzeichnet, daß $R_1$

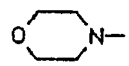

bedeutet.

5. Verfahren zur Herstellung eines pharmazeutischen Präparates, dadurch gekennzeichnet, daß ein Allylmercaptoacetyl-sydnonimin der allgemeinen Formel I oder ein pharmakologisch unbedenkliches Säureadditionssalz davon durch Mischen mit pharmazeutisch annehmbaren Träger- und Zusatzstoffen, gegebenenfalls in Kombination mit anderen pharmazeutischen Wirkstoffen in eine zur Verabreichung geeignete Form gebracht wird.

6. Verwendung der Allylmercaptoacetyl-sydnonimine der allgemeinen Formel I oder eines pharmakologisch unbedenklichen Säureadditionssalzes davon zur Herstellung von Arzneimitteln zur Behandlung cardiovakulärer Erkrankungen oder Angina pectoris.

## Claims

**Claims for the following Contracting States : AT, BE, CH, DE, FR, GB, IT, NL, SE**

1. Allylmercaptoacetylsydnonimines of the general formula I

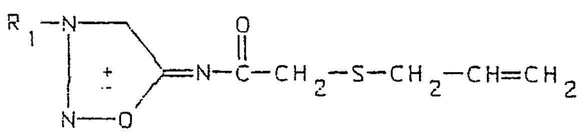

(I)

in which $R_1$ represents a secondary amino group of the formula

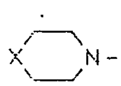

or

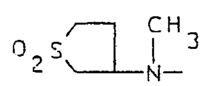

or $-N(R_3)_2$
and X denotes $NR_2$, $S(O)_n$, O or $(CH_2)_n$,
$R_2$ denotes one of the radicals $R_3OOC$-, $R_4SO_2$-, or alkyl having 1 - 4 C atoms,
n denotes one of the values 0, 1 or 2,
and $R_3$ denotes alkyl having 1 - 4 C atoms,
and $R_4$ represents $R_3$ or $(R_3)_2N$-,
and their pharmacologically acceptable salts.

2. Allylmercaptoacetylsydnonimines according to Claim 1, characterised in that $R_1$ denotes the radical

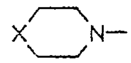

and X denotes $NR_2$, $SO_2$ or O and $R_2$ denotes $R_3OOC$- or $R_4SO_2$-, and $R_3$ denotes methyl or ethyl, and $R_4$ denotes methyl, ethyl, dimethylamino or diethylamino.

**3.** Allylmercaptoacetylsydnonimine according to Claim 1, characterized in that $R_1$ has the meaning

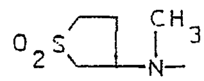

**4.** Allylmercaptoacetylsydnonimine of the formula I, characterized in that $R_1$ denotes

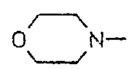

**5.** Process for the preparation of allylmercaptoacerylsydnonimine of the general formula I indicated in Claim 1, characterized in that, in a manner known per se,

a) a sydnonimine of the general formula II

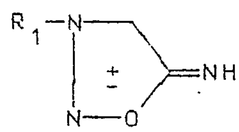

(II)

where appropriate in the form of a salt, is reacted with an acylating agent which introduces the allylmercaptoacetyl radical, or

b) a sydnonimine of the general formula II, where appropriate in the form of a salt, is acylated with an acetic acid derivative which contains a radical which is subsequently reacted with allyl mercaptan to give the allylmercapto compound of the general formula I.

**6.** Allylmercaptoacetylsydnonimines of the general formula I, or their pharmacologically acceptable acid additon salts, as pharmacological active compounds for the treatment of cardiovascular disorders or angina pectoris.

**7.** Pharmaceutical product, characterized in that it contains an allylmercaptoacetylsydnonimine of the general formula I, or a pharmacologically acceptable acid addition salt thereof, as active compound, together with pharmaceutically acceptable vehicles and additives, where appropriate in combination with other pharmaceutical active compounds.

**8.** Use of the allylmercaptoacetylsydnonimines of the general formula I, or of a pharmaceutically acceptable acid addition salt thereof, for the preparation of medicaments for the treatment of cardiovascular disorders or angina pectoris.

**Claims for the following Contracting States : ES, GR**

**1.** Process for preparing allylmercaptoacetylsydnonimines of the general formula I

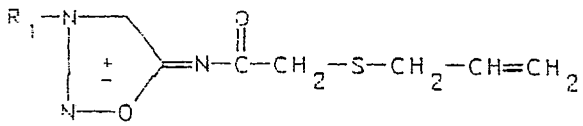

(I)

in which $R_1$ represents a secondary amino group of the formula

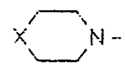

or

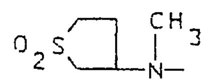

or $-N(R_3)_2$
and X denotes $NR_2$, $S(O)_n$, O or $(CH_2)_n$,
$R_2$ denotes one of the radicals $R_3OOC$-, $R_4SO_2$-, or alkyl having 1 - 4 C atoms,
n denotes one of the values 0, 1 or 2,
and $R_3$ denotes alkyl having 1 - 4 C atoms,
and $R_4$ represents $R_3$ or $(R_3)_2N$-,
and their pharmacologically acceptable salts, characterized in that, in a manner known per se,

a) a sydnonimine of the general formula II

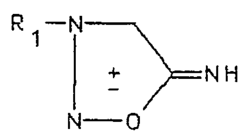

(II)

where appropriate in the form of a salt, is reacted with an acylating agent which introduces the allylmercaptoacetyl radical, or
b) a sydnonimine of the general formula II, where appropriate in the form of a salt, is acylated with an acetic acid derivative which contains a radical which is subsequently reacted with allyl mercaptan to give the allylmercapto compound of the general formula I.

**2.** Process according to Claim 1, characterized in that $R_1$ denotes the radical

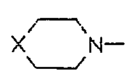

and X denotes $NR_2$, $SO_2$ or O and

$R_2$ denotes $R_3OOC$- or $R_4SO_2$-, and $R_3$ denotes methyl or ethyl, and $R_4$ denotes methyl, ethyl, dimethylamino or diethylamino.

**3.** Process according to Claim 1, characterized in that $R_1$ has the meaning

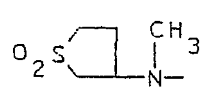

**4.** Process according to Claim 1, characterized in that $R_1$ denotes

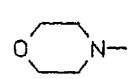

**5.** Process for preparing a pharmaceutical preparation, characterized in that an allylmercaptoacetylsydnonimine of the general formula I or a pharmacologically acceptable acid addition salt thereof is converted into a form suitable for administration by mixing with pharmaceutically acceptable vehicles and additives, where appropriate in combination with other pharmaceutical active compounds.

**6.** Use of the allylmercaptoacetylsydnonimines of the general formula I, or of a pharmacologically acceptable acid addition salt thereof, for the preparation of medicaments for the treatment of cardiovascular disorders or angina pectoris.

**Revendications**

**Revendications pour les Etats contractants suivants : AT, BE, CH, DE, FR, GB, IT, NL, SE**

**1.** Allylthioacétyl-sydnonimines répondant à la formule générale I

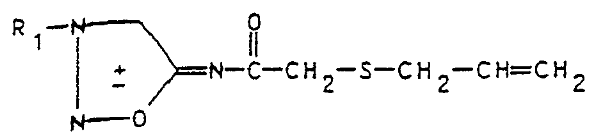 (I)

dans laquelle $R_1$ désigne un groupe amino secondaire répondant à la formule

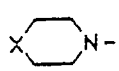 ou 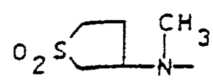

ou $N(R_3)_2$
et X représente $NR_2$, $S(O)_n$, O ou $(CH_2)_n$,
$R_2$ désigne un des radicaux $R_3OOC$-, $R_4SO_2$-, ou un groupe alkyle en $C_1$-$C_4$,
n est égal à 0, 1 ou 2,
$R_3$ est un groupe alkyle en $C_1$-$C_4$,
et $R_4$ est $R_3$ ou un groupe $(R_3)_2N$-,
ainsi que leurs sels pharmacologiquement acceptables.

**2.** Allylthioacétyl-sydnonimines selon la revendication 1, caractérisées en ce que $R_1$ désigne le radical

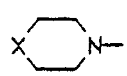

et X représente $NR_2$, $SO_2$, ou O, $R_2$ désigne un groupe $R_3OOC$- ou $R_4SO_2$-, $R_3$ un groupe méthyle ou éthyle, et $R_4$ un groupe méthyle, éthyle, diméthylamino ou diéthylamino.

**3.** Allylthioacétyl-sydnonimine selon la revendication 1, caractérisée en ce que $R_1$ désigne un groupe

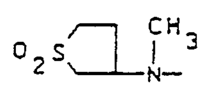

**4.** Allylthioacétyl-sydnonimine répondant à la formule 1, caractérisée en ce que $R_1$ désigne un groupe

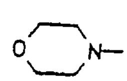

**5.** Procédé de préparation d'une allylthioacétyl-sydnonimine répondant à la formule générale indiquée

dans la revendication 1, caractérisé en ce que, d'une manière connue en soi,

a) on fait réagir une sydnonimine répondant à la formule générale II

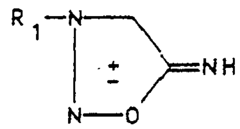

(II)

le cas échéant sous la forme d'un sel, avec un agent d'acylation introduisant le radical allylthioacétyle, ou

b) on acyle une sydnonimine répondant à la formule générale II, le cas échéant sous la forme d'un sel, avec un dérivé de l'acide acétique qui contient un radical que l'on met à réagir ensuite avec l' allylthiol pour donner le composé allylthio répondant à la formule générale I.

6. Allylthioacétyl-sydnonimines répondant à la formule générale I ou leurs sels d'addition aux acides pharmacologiquement acceptables en tant que principes actifs pharmacologiques dans le traitement de maladies cardiovasculaires ou de l'angine de poitrine.

7. Préparation pharmaceutique, caractérisée en ce qu'elle contient une allylthioacétyl-sydnonimine répondant à la formule générale I ou un de ses sels d'addition aux acides pharmacologiquement acceptables comme principe actif en même temps que des excipients et additifs pharmaceutiquement acceptables, le cas échéant en association avec d'autres principes actifs pharmaceutiques.

8. Utilisation d'allylthioacétyl-sydnonimines répondant à la formule générale I ou d'un de leurs sels d'addition aux acides pharmacologiquement acceptables pour la préparation de médicaments pour le traitement de maladies cardiovasculaires et de l'angine de poitrine.

**Revendications Pour les Etats contractants suivants : ES, GR**

1. Allylthioacétyl-sydnonimines répondant à la formule générale I

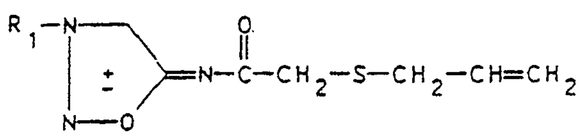

(I)

dans laquelle $R_1$ désigne un groupe amino secondaire répondant à la formule

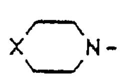

ou

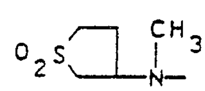

ou $N(R_3)_2$
et X désigne un groupe $NR_2$, $S(O)_n$, O ou $(CH_2)_n$,
$R_2$ désigne un des radicaux $R_3OOC$-, $R_4SO_2$-, ou un groupe alkyle en $C_1$-$C_4$,
n est égal à 0, 1 ou 2,
$R_3$ désigne un groupe alkyle en $C_1$-$C_4$,
et $R_4$ désigne $R_3$ ou un groupe $(R_3)_2N$-,
ainsi que leurs sels pharmacologiquement acceptables.

2. Allylthioacétyl-sydnonimines selon la revendication 1, caractérisées en ce que $R_1$ désigne le radical

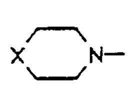

X désigne les radicaux NR$_2$, SO$_2$, ou O, R$_2$ les radicaux $R_3OOC$- ou $R_4SO_2$-, R$_3$ un radical méthyle ou éthyle, et R$_4$ un radical méthyle, éthyle, diméthylamino ou diéthylamino.

3. Allylthioacétyl-sydnonimine selon la revendication 1, caractérisée en ce que R$_1$ désigne un radical

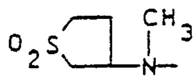

4. Allylthioacétyl-sydnonimine répondant à la formule 1, caractérisée en ce que R$_1$ désigne un radical

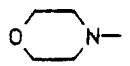 ,

5. Procédé de préparation d'une allylthioacétyl-sydnonimine répondant à la formule générale indiquée dans la revendication 1, caractérisé en ce que, d'une manière connue en soi,

a) on fait réagir une sydnonimine répondant à la formule générale II

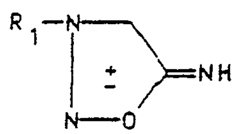

(II)

le cas échéant sous la forme d'un sel, avec un agent d'acylation introduisant le radical allylthioacétyle, ou

b) on acyle une sydnonimine répondant à la formule générale II, le cas échéant sous la forme d'un sel, avec un dérivé de l'acide acétique qui contient un radical que l'on peut faire réagir ensuite avec l'allylthiol pour donner le composé allylthio répondant à la formule générale I.

6. Procédé de confection d'une préparation pharmaceutique, caractérisé en ce qu'une allylthioacétyl-sydnonimine répondant à la formule générale I ou un de ses sels d'addition aux acides pharmacologiquement acceptables est amenée sous une forme appropriée pour l'administration par mélange avec des excipients et additifs pharmaceutiquement acceptables, le cas échéant en association avec d'autres principes actifs pharmaceutiques.